# FASCICULE DE BREVET EUROPEEN

(11) **EP 1 608 769 B1**
(45) Date de publication et mention de la délivrance du brevet: **11.01.2012**
(21) Numéro de dépôt: 04742828.9
(22) Date de dépôt: 02.04.2004
(51) Int. Cl.: C12Q 1/04, C12M 1/28, C12M 1/34, B01L 3/00

(54) **PROCEDE ET DISPOSITIF DE DETECTION ET/OU D'IDENTIFICATION DE BACTERIES PRESENTES DANS UN ECHANTILLON**
VERFAHREN UND VORRICHTUNG ZUM NACHWEISEN UND/ODER BESTIMMEN DER IN EINER PROBE ENTHALTENEN BAKTERIEN
METHOD AND DEVICE FOR THE DETECTION AND/OR IDENTIFICATION OF BACTERIA PRESENT IN A SAMPLE

(30) Priorité: 07.04.2003 FR 0304262
(43) Date de publication de la demande: 28.12.2005
(73) Titulaire: BIOMERIEUX, 69280 Marcy-L'Etoile (FR)
(72) Inventeur: MERCADER BADIA, Josep Vicent, 08014 Barcelona (ES); COLIN, Bruno, F-69280 Marcy L'Etoile (FR); ATRACHE, Vincent, F-69300 Caluire (FR)
(74) Mandataire: Bitaud, Valérie Marie-Odile
(86) Numéro de dépôt international: PCT/FR2004/050140
(87) Numéro de publication internationale: WO 2004/092401

(56) Documents cités:
- EP-A- 0 259 116
- WO-A-00/63668
- DE-A- 19 605 753

## Description

La présente invention concerne un procédé de détection et/ou d'identification de bactéries présents dans un échantillon, liquide ou solide. L'invention concerne également un dispositif de détection et/ou d'identification de bactéries présents dans un tel échantillon.

L'identification de bactéries dans les aliments, est indispensable pour la santé publique. Par exemple, les bactéries de genre *Salmonella,* qui peuvent être trouvées dans une grand nombre d'aliments, sont la cause de nombreuses pathologies chez l'homme (fièvre typhoïde, intoxication alimentaire). De même, l'isolement et l'identification de la bactérie *Listeria* est une obligation majeure de la surveillance de l'hygiène agro-alimentaire et de la bactériologie médicale. Ainsi, parmi les bactéries du genre *Listeria,* l'espèce *Listeria monocytogenes,* connue pour être pathogène pour l'homme peut engendrer la listériose, parfois mortelle (25 à 30 % des cas) chez les personnes immunodéprimées, les enfants en bas âge. Il est donc très important de disposer d'un test fiable et rapide permettant de détecter les contaminations par ces bactéries, lequel test doit être à la fois sensible et spécifique. Enfin, l'identification de bactéries du genre Staphylocoque est également primordial. La plupart de ces espèces sont des pathogènes opportunistes chez l'homme présentant un risque élevé en cas de blessure cutanée par un traumatisme ou par implantation directe d'un produit médical. D'ailleurs, l'espèce *Staphylococcus aureus* est une bactérie qui se retrouve souvent chez des patients qui doivent recevoir des soins hospitaliers faisant intervenir des appareils tels que seringues ou cathéters. Il y a donc un grand intérêt de détecter h présence de cette bactérie pathogène, de plus en plus impliquée dans les maladies nosocomiales.

Il existe actuellement de nombreux tests de détection de bactéries. D'une manière générale, ces tests de détection nécessite un préenrichissement comprenant :
∘ une étape de ressuscitation, permettant aux bactéries de récupérer du stress induit par la mise en culture et
∘ une étape de croissance exponentielle bactérienne, qui est indispensable lorsque peu de bactéries sont initialement présent dans l'échantillon et
∘ une étape de détection des bactéries, qui doit être préférentiellement réalisée au pic de croissance bactérienne, et avant l'apparition de la phase de mort bactérienne, qui est observée lors de toute culture bactérienne.
A titre indicatif de méthode de détection de bactéries, on peut citer le procédé de détection de *Salmonella* décrit dans le brevet US-A-4,563,418, qui est basé sur une immunoprécipitation. La détection de ces bactéries s'effectue, après l'étape de pré enrichissement et de croissance bactérienne, après migration dans un milieu de culture pré enrichi en *Salmonella* et une réaction avec des anticorps dirigés contre les *Salmonella.* On peut citer également une méthode comparable, basée également sur une immunoprécipitation qui est présentée dans le brevet US-A-5,132,229. Dans ces méthodes, l'utilisateur doit toutefois réalisée manuellement une étape de transfert du milieu de culture, pré enrichi en bactéries, vers le gel de migration, ce qui peut induire des problèmes notamment de contamination mais également des risques pour la santé du manipulateur.

On peut également citer des méthodes plus récentes, basées principalement sur la migration des bactéries à travers un milieu sélectif de l'espèce de bactéries que l'on souhaite spécifiquement détecter. Ainsi, la demande de brevet EP-A-0.259.116 présente un procédé pour détecter la présence de *Salmonella* dans un échantillon, le procédé comprenant la migration des *Salmonella* présentes dans un milieu de culture pré enrichi vers un milieu sélectif, spécifique des salmonelles. Les salmonelles migrent alors vers le système de détection, permettant leur identification, alors que les autres bactéries susceptibles d'être présents dans l'échantillon sont stoppés par le milieu sélectif. Toutefois, dans ce procédé, le milieu de culture reste en contact avec le milieu sélectif et le système de détection, ce qui constitue le problème majeur de ce type de procédé. Ainsi, si l'échantillon est fortement contaminé, les bactéries, autres que les salmonelles présentes dans le milieu de culture, peuvent alors saturer le milieu sélectif, qui ne joue plus alors son rôle de barrière sélective. La présence de bactéries, autres que les salmonelles, dans le système de détection peut alors constituer un faux positif: on détecte un système de détection positif alors que l'échantillon n'est pas contaminé en salmonelles. De plus, le procédé tel que décrit dans cette demande ne peut être mis en oeuvre que pour des bactéries motiles.

La présente invention se propose de résoudre l'ensemble des inconvénients de l'état de la technique en présentant notamment un procédé pour la détection et/ou l'identification de bactéries, nécessitant peu de manipulations de la part du manipulateur, du milieu de culture comprenant les bactéries, tout en permettant un diagnostic rapide, directement depuis le milieu de culture, que l'on peut mettre aisément en place lors de la phase de croissance exponentielle bactérienne.

Avant d'aller plus avant, les définitions suivantes permettront de mieux comprendre la suite de l'exposé de l'invention.
Par échantillon, on entend un échantillon alimentaire, issu de tout type d'aliment, mais aussi un échantillon clinique, issu d'un prélèvement de liquide biologique. Cet échantillon peut être ainsi liquide ou solide et on peut citer d'une manière non limitative, un échantillon alimentaire d'eau, de boissons tels que le lait, un jus de fruits; échantillon clinique de sang, de plasma, d'urines, de fécès, un échantillon alimentaire de yaourt, de viande, d'oeufs, légumes, mayonnaise, fromage ; poisson..., un échantillon alimentaire issue d'une alimentation destinée aux animaux, tel que notamment un échantillon issu de farines animales
Par premier récipient, on entend tout type de contenant susceptible de recevoir un milieu de culture, tel que notamment un flacon, une bouteille, un sac Stomacher®... Il peut être avantageux que ce premier récipient tolère une variation de pression, lors notamment d'une variation de température. Ainsi, si le premier récipient est fabriqué dans un matériau solide (telle qu'une bouteille en verre), il est avantageux que ce premier récipient soit ouvert, ou soit munie d'une valve permettant une variation de pression. Lorsque le premier récipient est fabriqué dans un matériau flexible, tel que notamment, un sac Stomacher®, il peut être ouvert ou fermé, puisque la variation de pression est permise par la flexibilité du matériau.
Par milieu de culture, on entend un milieu comprenant tous les éléments nécessaires à la viabilité et à la croissance des cellules. Un tel milieu de culture est bien connu de l'homme du métier, qui pourra aisément choisir le milieu de culture le plus adéquat quant aux bactéries qu'il souhaite détecter.
Par milieu sélectif, on entend un milieu permettant de sélectionner les bactéries qui doivent réagir avec le système de détection des bactéries. Lorsque l'on souhaite sélectionner par exemple une bactérie donnée, le milieu sélectif comprend un antibiotique dirigé contre les autres espèces bactériennes, susceptibles d'être présentes dans l'échantillon, et que l'on ne veut pas détecter. Un autre exemple consisterait en ce que ce milieu sélectif comprend un composé toxique contre certains bactéries particuliers. On peut utiliser encore un milieu restrictif nutritionnellement pour certaines bactéries. De tels milieux sélectifs sont bien connus de l'homme du métier qui choisira un milieu sélectif approprié en fonction de l'échantillon et des bactéries qu'il souhaite analyser. Ce milieu sélectif peut être mélangé au milieu de culture ou isolé dans un deuxième récipient comprenant le système de détection.
Par deuxième récipient, on entend tout type de contenant susceptible de recevoir au moins un système de détection tel que défini ci-dessous. Ce deuxième récipient est préférentiellement dans un matériau suffisamment rigide, pour que son volume reste constant lorsqu'on applique à l'intérieur de ce deuxième récipient, une diminution de température. Ce deuxième récipient peut être compris dans le premier récipient afin que l'utilisateur ne dispose que d'un seul dispositif comprenant l'ensemble des moyens nécessaire à la détection et/ou à l'identification de bactéries.
Par système de détection, on entend un système qui permet de mettre en évidence la présence d'un bactérie donné. Un tel système est bien connu de l'homme du métier et on peut citer, à titre indicatif, un système de détection comprenant un substrat chromogénique, fluorogénique, basé sur la détection d'une immunoprécipitation, une immunochromatographie, un indicateur de pH, un produit précipitant... Cette détection peut être directe, c'est-à-dire par détection du bactérie, ou indirecte, par détection notamment d'un métabolite libéré par ledit bactérie. Par moyen de transfert, on entend tout moyen permettant le transfert d'un milieu liquide compris dans un premier récipient vers un deuxième récipient Ce moyen de transfert et le deuxième récipient peuvent être indépendant, ou constituer une pièce unique.
Ainsi, l'invention présente un procédé de détection et/ou d'identification de bactéries présents dans un échantillon, liquide ou solide, caractérisé en ce que :
a. on place, dans un premier récipient, l'échantillon susceptible de contenir lesdites bactéries dans un milieu de culture liquide,
b. on dispose d'un deuxième récipient comprenant au moins un système de détection desdites bactéries,
c. on dispose d'un moyen de transfert entre le premier récipient et le deuxième récipient, ledit moyen de transfert comprenant au moins une première ouverture au niveau du premier récipient et au moins une deuxième ouverture au niveau du deuxième récipient, de telle façon que le deuxième récipient circonscrit un premier volume d'air entre l'ouverture du moyen de transfert et le système de détection desdites bactéries
d. on applique une température T1 à l'intérieur du deuxième récipient puis,
e. on applique une température T2 à l'intérieur du deuxième récipient
f. la température T1 est supérieure à la température T2 de sorte que l'on transfert un volume défini de milieu de culture du premier récipient vers le deuxième récipient ,
g. on détermine la présence ou l'absence de bactéries et/ou on identifie les bactéries au niveau du système de détection.
Les bactéries peuvent être notamment d'une manière non limitative les bactéries du genre *Staphylococcus, Salmonella, Niesseria, Legionella, Mycobacteria, Campylobacter, Listeria...*

Il est bien entendu que le transfert entre le premier récipient et le dixième récipient ne s'effectue pas spontanément Il faut par exemple que la première ouverture au niveau du premier récipient soit situé au dessous de la deuxième ouverture au niveau du deuxième récipient, afin d'éviter tout transfert spontanée par gravité. Ce transfert spontané peut être évité également lorsque le moyen de transfert est un conduit non capillaire, empêchant tout transfert spontanée entre le premier récipient et le deuxième récipient par simple capillarité. Le diamètre interne de ce conduit peut être compris notamment entre 0,1 et 5 mm, préférentiellement entre 0,5 et 2 mm.

Préférentiellement, le moyen de transfert circonscrit un deuxième volume d'air entre la première ouverture et la deuxième ouverture. Si le deuxième récipient comprend en outre, entre la deuxième ouverture du moyen de transfert et le système de détection, un milieu sélectif, le volume d'air est préférentiellement circonscrit entre la deuxième ouverture du moyen de transfert et le milieu sélectif

Selon un mode préféré de réalisation de l'invention, le deuxième récipient et le moyen de transfert sont dans des matériaux suffisamment rigides pour rester à un volume constant lors d'une variation de température. Ainsi, lorsqu'on passe d'une température T1 à T2, le premier volume d'air, et/ou le deuxième volume d'air agissent comme un poumon d'aspiration en subissant une diminution de pression.

Selon un mode préféré de réalisation de l'invention, T1 est compris entre 20 et 45°C, et plus préférentiellement entre 30 et 42°C ; T2 est compris entre 4 et 30 °C, préférentiellement entre 13 et 18°C.

Lors de l'étape f) le volume défini que l'on transfert est notamment dépendant de la différence de température que l'on applique, ainsi que du deuxième volume d'air. Le volume défini que l'on transfert peut être notamment compris entre 100 et 1000 µl, et préférentiellement entre 400 et 600 µl. Ainsi, a titre indicatif, lorsque la différence de température (T2 - T1) est de 22°C, et que T1 = 15°C et T2 = 37°C, une volume de 350 µl est transféré du premier récipient vers le deuxième récipient Il est également possible de réaliser les étapes d) à f) plusieurs fois, afin de transférer, en plusieurs fois, le volume défini permettant la détection des bactéries.

Lors de l'étape g), la détermination de la présence ou l'absence de bactéries et/ou l'identification des bactéries dépend du système de détection qui est utilisé.

Selon une variante de réalisation de l'invention, on effectue, entre l'étape a) et l'étape b), un étape de pré enrichissement qui consiste à augmenter le nombre de bactéries que l'on souhaite détecter. Une telle étape de pré enrichissement est bien connue de l'homme du métier, qui l'adaptera aisément selon les bactéries qu'il souhaite détecter.

Selon un mode particulier de réalisation de l'invention, le deuxième récipient est compris dans le premier récipient

L'invention concerne également un procédé de détection et/ou d'identification de bactéries présentes dans un échantillon, liquide ou solide, caractérisé en ce que :
a. on place, dans un premier récipient, l'échantillon susceptible de contenir lesdites bactéries dans un milieu de culture liquide, tel que défini précédemment,
b. on dispose d'un deuxième récipient comprenant au moins un système de détection desdites bactéries, tel que défini précédemment,
c. on dispose d'un moyen de transfert entre le premier récipient et le deuxième récipient, tel que défini précédemment,
d. on applique une température T1 à l'intérieur du premier récipient puis,
e. on applique une température T2 à l'intérieur du premier récipient
f. la température T1 est inférieure à la température T2 de sorte que l'on transfert un volume défini de milieu de culture du premier récipient vers le deuxième récipient ,
g. on détermine la présence ou l'absence de bactéries et/ou on identifie les bactéries au niveau du système de détection.

Selon un mode préféré de réalisation de l'invention, le deuxième récipient et le moyen de transfert sont dans des matériaux suffisamment rigides pour rester à un volume constant lors d'une variation de température.

L'invention concerne également un dispositif de détection et/ou d'identification de bactéries dans un échantillon comprenant
- un premier récipient,
- un deuxième récipient comprenant au moins un système de détection, et
- au moins un moyen de transfert entre un premier récipient et le deuxième récipient, ledit moyen de transfert comprenant au moins une première ouverture au niveau du premier récipient et au moins une deuxième ouverture au niveau du deuxième récipient ; ledit deuxieme récipient circonscrivant un premier volume d'air entre ledit systeme de detection et la deuxième ouverture du moyen de transfert.

Selon un mode préféré de réalisation de l'invention, le moyen de transfert circonscrit un deuxième volume d'air entre la première ouverture et la deuxième ouverture, lesdits premier et deuxième volumes d'air agissant comme un poumon d'aspiration lorsqu'on applique une diminution de température au sein notamment du deuxième récipient. Selon un mode préféré de réalisation de l'invention, le premier récipient et le moyen de transfert sont dans des matériaux suffisamment rigides pour rester à un volume constant lors d'une variation de température.

Selon un mode particulier de réalisation de l'invention, la première ouverture au niveau du premier récipient est en position inférieure par rapport à la deuxième ouverture au niveau du deuxième récipient, afin d'éviter notamment tout transfert par pesanteur entre le premier récipient et le deuxième récipient.

Selon un autre mode de réalisation de l'invention, la première ouverture au niveau du premier récipient et la deuxième ouverture au niveau du deuxième récipient constitue la même ouverture. Dans ce mode de réalisation, le moyen de transfert se limite alors à cette même ouverture.

Selon un mode particulier de réalisation de l'invention, le moyen de transfert est un conduit non capillaire, afin d'éviter notamment tout transfert par simple capillarité entre le premier récipient et le deuxième récipient

Selon un mode particulier de réalisation de l'invention, le dispositif tel que défini ci dessus comprend, en outre, un premier récipient comprenant un milieu de culture. Selon un mode particulier, le deuxième récipient est compris dans le premier récipient Préférentiellement, le premier récipient est un sac stomacher®. Le sac stomacher® peut alors comprendre un filtre, entre le milieu de culture ou est placé l'échantillon alimentaire, et le moyen de transfert, afin d'éviter que des fragments de l'échantillon alimentaire ne bouche la première ouverture et empêche le transfert du milieu de culture du premier récipient vers le deuxième récipient.

L'invention concerne enfin un kit de détection et/ou d'identification de bactéries comprenant un dispositif tel que défini précédemment.

Les figures ci-jointes sont données à titre d'exemple explicatif et n'ont aucun caractère limitatif. Elles permettront de mieux comprendre l'invention.

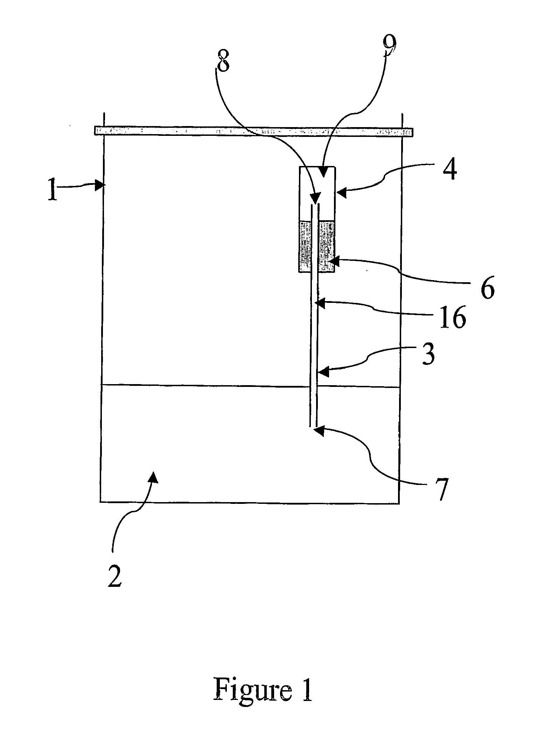

Ainsi, la figure 1 présente un premier mode de réalisation de l'invention, tel que présenté dans l'exemple 1. Le premier récipient (1) est un sac stomacher® comprenant un milieu de culture (2) dans lequel est placé l'échantillon, susceptible de contenir les bactéries que l'on souhaite détecter et/ou identifier. Le deuxième récipient (4) comprend un système de détection (6) qui est un milieu liquide sélectif comprenant un indicateur formant un précipité lorsqu'il est en présence de la bactérie que l'on souhaite détecter et/ou identifier. Ce deuxième récipient est relié à un moyen de transfert (3), qui plonge dans le milieu de culture (2) comprenant l'échantillon. Ce moyen de transfert comprend une première ouverture (7) au niveau du premier récipient (1) et une deuxième ouverture (8) au niveau du deuxième récipient (4). Un premier volume d'air (9) est circonscrit entre la deuxième ouverture (8) et le système de détection (6). Un deuxième volume d'air (16) est également circonscrit au sein du moyen de transfert (3) entre la deuxième ouverture (8) et la première ouverture (7).

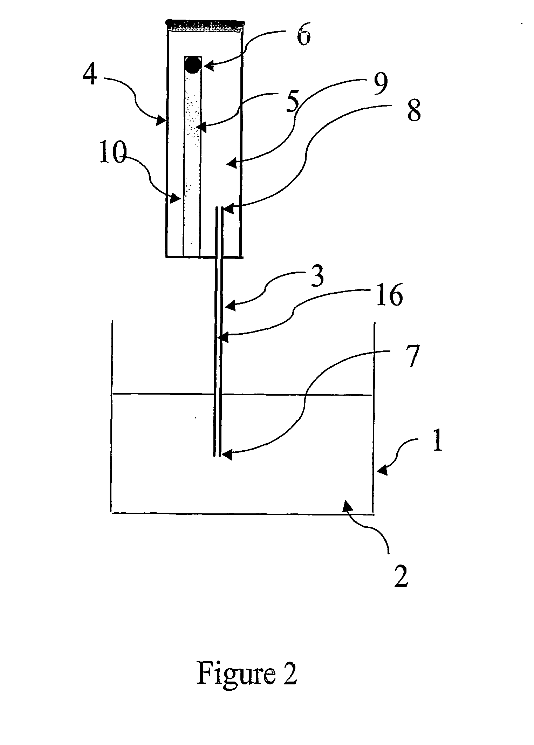

La figure 2 présente un autre mode de réalisation de l'invention, tel que présenté dans l'exemple 2. Le premier récipient (1) est un contenant ouvert comprenant un milieu de culture (2) dans lequel est placé l'échantillon que l'on souhaite analyser. Le moyen de transfert (3) est comparable à celui de la figure 1. Le deuxième récipient (4) comprend un premier volume d'air (9), tel que défini dans la figure 1, et une colonne de cellulose (10) dans laquelle est imbibé un milieu sélectif (5). Au somment de la colonne de cellulose (10) est placé le système de détection (6) qui est un substrat chromogénique immobilisé passivement sur un disque de papier stérile.

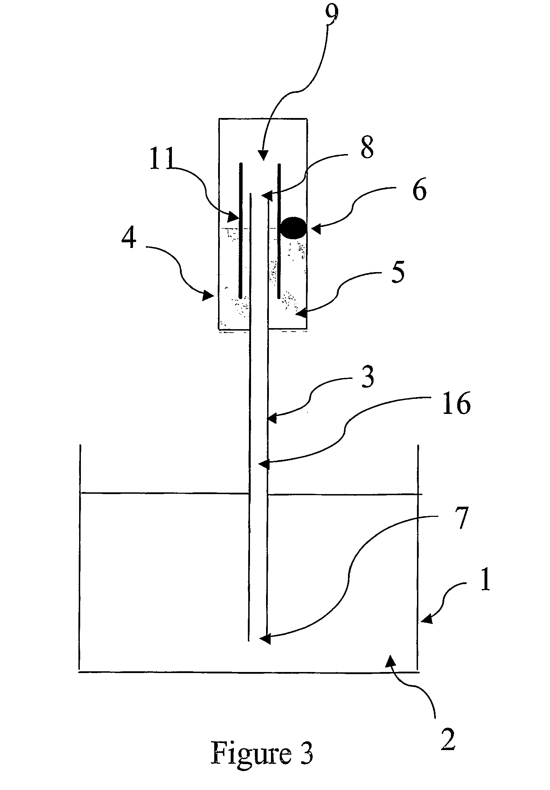

La figure 3 présente un autre mode de réalisation de l'invention, tel que présenté dans l'exemple 3, qui trouve une application préférentielle lors de la détection de bactéries motiles. Le premier récipient (1) est un contenant ouvert comprenant un milieu de culture (2) dans lequel est placé l'échantillon que l'on souhaite analyser. Le moyen de transfert (3) est comparable à celui de la figure 1. Le deuxième récipient (4) comprend un premier volume d'air (9) tel que défini dans la figure 1, un milieu sélectif (5) semi solide, et un système de détection (6) qui est un substrat chromogénique immobilisé passivement sur un disque de papier stérile. Un cylindre (11) contraint, après l'étape de transfert, les bactéries motiles à migrer à travers le milieu sélectif avant d'atteindre le système de détection.

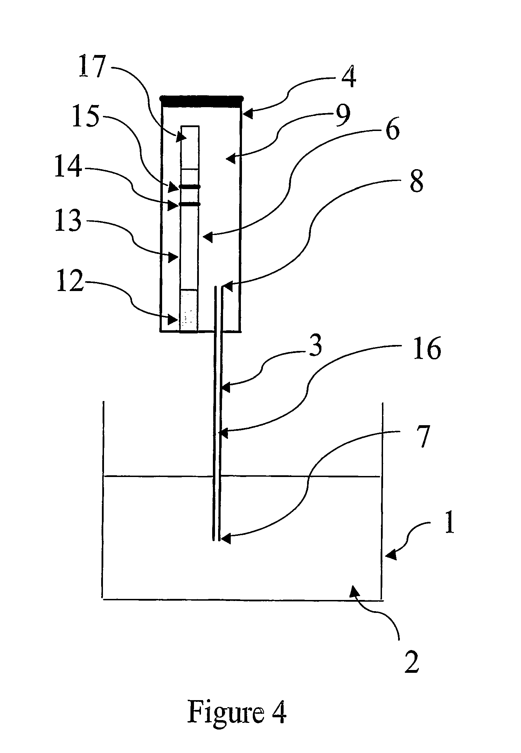

La figure 4 présente un autre mode de réalisation de l'invention, tel que présenté dans l'exemple 4. Le premier récipient (1) est un contenant ouvert comprenant un milieu de culture (2) dans lequel est placé l'échantillon que l'on souhaite analyser. Le moyen de transfert (3) est comparable à celui de la figure 1. Le deuxième récipient (4) comprend un premier volume d'air (9) tel que défini dans la figure 1, et un système de détection (6) qui est une bandelette d'immunochromatographie, bien connue de l'homme du métier, comprenant une première partie (12) contenant un conjugué anticorps anti bactéries marqué, une deuxième partie (13) qui est une membrane de chromatographie, sur laquelle on peut détecter un premier signal (14) caractéristique de la présence des anticorps anti bactéries que l'on souhaite détecter, et un deuxième signal (15), caractéristique de la présence d'un témoin positif. Le papier absorbant (17) permet la migration des bactéries, même si elles ne sont pas motiles.

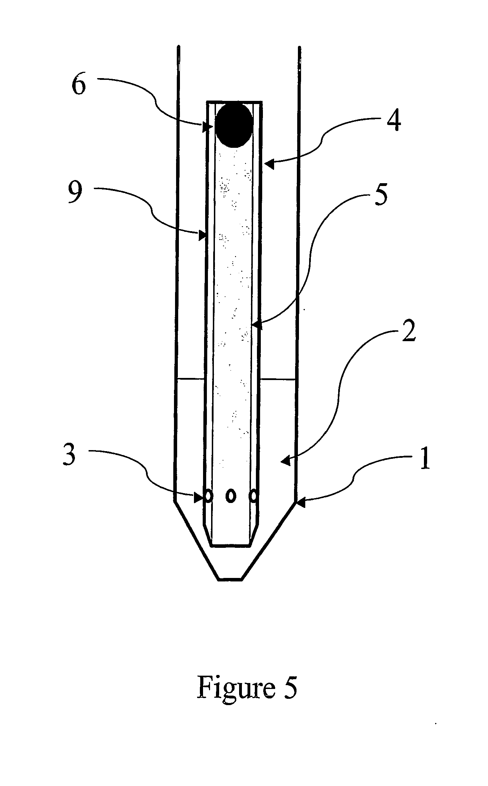

La figure 5 présente un autre mode de réalisation de l'invention. Le premier récipient (1) est un tube d'analyse biologique comprenant un milieu de culture (2) dans lequel est placé l'échantillon que l'on souhaite analyser. Le deuxième récipient (4) comprend un premier volume d'air (9) tel que défini dans la figure 1, et un système de détection (6) et un milieu sélectif (5). Dans ce mode de réalisation, la première ouverture au niveau du premier récipient (1) et la deuxième ouverture au niveau du deuxième récipient (4) constitue la même ouverture : le moyen de transfert (3) se limite alors à cette même ouverture. Cette ouverture ne doit pas permettre le transfert spontané de milieu de culture entre le premier et le deuxième récipient.

Les exemples suivants sont donnés à titre illustratif et n'ont aucun caractère limitatif. Ils permettront de mieux comprendre l'invention.

### Exemple 1- Détection de Staphylococcus aureus sur un milieu sélectif liquide comprenant un indicateur

Un dispositif de détection et/ou d'identification de bactéries présent dans un échantillon tel que présenté dans la figure 1 est utilisé dans cet exemple pour la détection de bactéries *Staphylococcus aureus.*

Pour cela, 225 ml de milieu de culture (2) (eau peptonée, bioMérieux, ref 42043) est introduit dans premier récipient (1) qui est un sac stomacher®, et 25 ml de lait, et le mélange est inoculé avec 3x10⁴ cellules *Staphylococcus aureus,* selon un protocole classique tel que connu de l'homme du métier. Un test contrôle est obtenu par l'utilisation de milieu de culture et de lait, tel que présenté ci dessus, mais non inoculé par *Staphylococcus aureus.* La partie supérieure d'une seringue de 25 ml est utilisé comme deuxième récipient (4) comprenant un premier volume d'air (9) de 13 ml. Un milieu sélectif (5) (Qi-Staph®, bioMérieux) est utilisé, et 3 ml de ce milieu sélectif sont introduit à l'intérieur du deuxième récipient (4). Dans cet exemple, le système de détection (6) est un composé-indicateur (tellurite de potassium) qui permet l'apparition d'un précipité noir en présence de bactéries *Staphylococcus aureus.* Le moyen de transfert (3) utilisé dans cet exemple est un conduit, d'un diamètre interne de 1 mm et une longueur de 12 cm, dont 4 cm pénètrent à l'intérieur du deuxième récipient (4), tel que présenté dans la figure 1. Ce moyen de transfert (3) comprend une première ouverture (7) qui est immergé dans le milieu de culture (2), et une deuxième ouverture (8) qui rentre à l'intérieur du deuxième récipient (4). Dans cet exemple, le deuxième récipient ne comprend qu'une seule ouverture, qui correspond à ladite deuxième ouverture (8) du moyen de transfert (3), et constitue de ce fait une enceinte fermée à l'exception de l'ouverture (8). Comme présenté dans la figure 1, le deuxième récipient (4) et le moyen de transfert (3) sont placés dans le sac stomacher®. Ceci permet d'isoler le milieu de culture contaminé.

Le transfert du milieu de culture (2) comprenant les bactéries entre le premier récipient (1) et le deuxième récipient (4) est obtenu en plaçant l'ensemble du dispositif dans un incubateur à 37°C pendant une heure. Cette incubation permet le pre enrichissement du milieu de culture. Une incubation plus ou moins longue peut être réalisée selon la contamination de l'échantillon. L'intérieur du deuxième récipient (4) est alors à une température T1 = 37°C. Puis, l'ensemble du dispositif est placé à une température de 15°C pendant une heure, afin que l'intérieur du deuxième récipient (4) soit à une température T2 = 15°C. Cette diminution de température induit alors une diminution de pression au sein du deuxième récipient (4), qui permet le transfert d'un volume défini du milieu de culture (600 µl) du premier récipient (1) vers le deuxième récipient (4). L'ensemble du dispositif est ensuite maintenu durant 18h à 37°C, afin de permettre aux bactéries de métaboliser le substrat, induisant l'apparition d'un précipité au niveau du système de détection (6). A noter que dans le deuxième récipient (4), le niveau de milieu sélectif (5) n'atteint pas la deuxième ouverture (8), entre le moyen de transfert (3) et le deuxième récipient (5) : lors de l'aspiration, le milieu de culture (2) comprenant les bactéries *Staphylococcus aureus* aspiré est piégé à l'intérieur du deuxième récipient (4), et ne peut refouler dans le premier récipient (1), lorsque qu'on augmente de nouveau la température à l'intérieur du deuxième récipient (4). A la fin de cette incubation, un précipité apparaît au niveau du système de détection, confirmant la présence de *Staphylococcus aureus* lorsque le milieu de culture et le lait était inoculé par ces bactéries. Aucun précipité n'était observé dans le test contrôle.

### Exemple 2 - Détection de bactéries Salmonella thyphimurium sur une colonne de cellulose

Le premier récipient (1) est identique à celui décrit dans l'exemple 1, mais pourrait être tout type de premier récipient. Dans cet exemple, 225 ml de milieu de culture (2) BPW (eau peptonée) dans lequel sont dissous 25g de poudre de lait (issue du commerce) sont inoculés avec 3x10² cellules *Salmonella typhimurium.* Le deuxième récipient (4) et le moyen de transfert (3) utilisé dans cet exemple est présenté dans la figure 2 . Un test contrôle est réalisé tel que décrit dans l'exemple 1.

Pour le milieu sélectif (5), 0,5 millilitre de milieu de culture BPW contenant 20 µg de novobiocine sont imbibés sur une colonne de cellulose (10) de 2 cm de long et 0,7 cm de diamètre. Cette colonne de cellulose (10) est placé à l'intérieur du deuxième récipient (4), sans toutefois être en contact avec la deuxième ouverture (8) du moyen de transfert (3). Dans cet exemple, le deuxième récipient (4) ne comprend qu'une seule ouverture, qui correspond à ladite deuxième ouverture (8) du moyen de transfert (3), et constitue de ce fait une enceinte fermée à l'exception de l'ouverture (8). En haut de cette colonne de cellulose (10) est déposé le système de détection (6) qui est un substrat chromogénique, le 5-bromo-6-chloro-3-indoxyl octanoate (magenta C8) combiné à du nitro bleu tetrazolium (Sigma, N6639), immobilisé passivement sur un disque de papier stérile d'un diamètre de 5 mm (Wathman 3 Chr, ref 3030917). Le transfert du milieu de culture (2) comprenant les bactéries entre le premier récipient (1) et le deuxième récipient (4) est réalisé tel que décrit dans l'exemple 1, en plaçant l'ensemble du dispositif à 37°C pendant 1 heure, puis à 15°C pendant une heure. A la fin du transfert, les bactéries, en contact avec la colonne de cellulose diffusent jusqu'en haut de la colonne, vers le système de détection où elles métabolisent le substrat, pour former un précipité noir. Les résultats positifs sont observé après 15h. Les résultats étaient négatifs lors du test contrôles.

### Exemple 3 - Détection de bactéries motiles Salmonella thyphimurium sur un milieu sélectif semi solide

Le premier récipient (1) est à celui décrit dans l'exemple 1, mais peut être tout type de premier récipient. Dans cet exemple, 225 ml de milieu de culture (2) BPW (eau peptonée), dans lequel sont dissous 25g de poudre de lait (issue du commerce) sont inoculés avec 3x10⁴ cellules *Salmonella thyphimurium.* Le deuxième récipient (4) et le moyen de transfert (3) utilisé dans cet exemple est présenté dans la figure 3. Un test contrôle est réalisé tel que décrit dans l'exemple 1.

A l'intérieur du deuxième récipient (4) sont déposés 3 ml de milieu semi solide (API M-medium®, bioMérieux, ref 50120) et utilisés comme milieu sélectif (5) comprenant 40 µg/ml de novobiocine, sans que le niveau de milieu sélectif (5) n'atteigne la deuxième ouverture (8), entre le moyen de transfert (3) et le deuxième récipient (5). La deuxième ouverture (8) et le système de détection (6) sont séparés par un cylindre (11) tel que présenté dans la figure 3 obligeant les bactéries à migrer à travers le milieu sélectif (5), ce qui fait que seules les bactéries résistantes et motiles parviennent au système de détection (6). Le système de détection (6) est un substrat chromogénique tel que décrit dans l'exemple 2.

Le transfert de salmonelles du premier récipient vers le deuxième récipient est réalisée, d'une façon comparable à l'exemple 1, en plaçant l'ensemble du dispositif à 37°C pendant 1 heure, puis à 15°C pendant une heure.

A la fin de l'étape de transfert, en maintenant l'ensemble du dispositif durant 16h à 37°C, un volume défini de milieu de culture (150 µl) comprenant les bactéries motiles est en contact avec le milieu sélectif (5), et les bactéries migrent à travers lui, jusqu'au système de détection (6). A la fin de cette incubation, la coloration du disque en noir confirme la présence de salmonelles, initialement présente dans le milieu de culture. Aucun coloration n'était observée lors du test contrôle.

### Exemple 4 - Détection de bactéries Listeria monocytogenes par immunochromatographie

Le premier récipient (1) est à celui décrit dans l'exemple 1, mais peut être tout type de premier récipient. Dans cet exemple, 250 ni de milieu de culture (2) (trypcase soya broth, bioMérieux, ref 44011 ; 6% de levure), sont inoculé avec 3x10⁷ cfu/ml de *Listeria monocytogenes 4b.* Le deuxième récipient (4) et le moyen de transfert (3) utilisé dans cet exemple est présenté dans la figure 4. Un test contrôle est réalisé d'une façon comparable mais sans inoculation par des bactéries.

A l'intérieur du deuxième récipient (4) est placé le système de détection (6) qui est une bandelette d'immunochromatographie bien connu de l'homme du métier. Un test contrôle est réalisé tel que décrit dans l'exemple 1.

Le transfert de *Listeria monocytogenes* du premier récipient (1) vers le deuxième récipient (4) est réalisée par l'incubation de l'ensemble du dispostif à une température T1 = 30 °C pendant une heure, puis une température T2 =15°C pendant 45 min. A la fin du transfert, on peut détecter un premier signal (14) caractéristique de la présence d'une protéine présent dans le flagelle de *L. monocytogenes* par réaction avec des anticorps spécifiques et un deuxième signal (15), caractéristique de la présence d'un témoin positif et obtenu par l'utilisation d'anticorps anti IgG de souris. Lors du test contrôle, seul le deuxième signal pouvait être observé.

### REFERENCES

- 1.: premier récipient
- 2.: milieu de culture comprenant l'échantillon
- 3.: moyen de transfert
- 4.: deuxième récipient
- 5.: milieu sélectif
- 6.: système de détection
- 7.: première ouverture
- 8.: deuxième ouverture
- 9.: premier volume d'air
- 10.: colonne de cellulose
- 11.: cylindre
- 12.: première partie
- 13.: deuxième partie
- 14.: premier signal
- 15.: deuxième signal
- 16.: deuxième volume d'air
- 17.: papier absorbant

## Revendications

1. Procédé de détection et/ou d'identification de bactéries présentes dans un échantillon, liquide ou solide, **caractérisé en ce que** :
a. on place, dans un premier récipient (1), l'échantillon susceptible de contenir lesdites bactéries dans un milieu de culture (2) liquide,
b. on dispose d'un deuxième récipient (4) comprenant au moins un système de détection (6) desdites bactéries,
c. on dispose d'un moyen de transfert (3) entre le premier récipient (1) et le deuxième récipient (4), ledit moyen de transfert (3) comprenant au moins une première ouverture (7) au niveau du premier récipient (1) et au moins une deuxième ouverture (8) au niveau du deuxième récipient (4), de telle façon que le deuxième récipient (4) circonscrit un premier volume d'air (9) entre l'ouverture (8) du moyen de transfert (3) et le système de détection (6) desdites bactéries,
d. on applique une température T1 à l'intérieur du deuxième récipient (4) puis,
e. on applique une température T2 à l'intérieur du deuxième récipient (4)
f. la température T1 est supérieure à la température T2 de sorte que l'on transfert un volume défini de milieu de culture (2) du premier récipient (1) vers le deuxième récipient (4),
g. on détermine la présence ou l'absence de bactéries et/ou on identifie les bactéries au niveau du système de détection (6).

2. Procédé selon la revendication 1 **caractérisé en ce que** le moyen de transfert (3) circonscrit un deuxième volume d'air (16) entre la première ouverture (7) et la deuxième ouverture (8).

3. Procédé selon l'une des revendications 1 ou 2 **caractérisé en ce que** T1 est compris entre 25 et 45°C, préférentiellement entre 30 et 42°C

4. Procédé selon l'une quelconques des revendications 1 à 3 **caractérisé en ce que** T2 est compris entre 4 et 24 °C, préférentiellement entre 13 et 18°C.

5. Procédé de détection et/ou d'identification de bactéries présentes dans un échantillon, liquide ou solide, **caractérisé en ce que** :
a. on place, dans un premier récipient (1), l'échantillon susceptible de contenir lesdites bactéries dans un milieu de culture (2) liquide,
b. on dispose d'un deuxième récipient (4) comprenant au moins un système de détection (6) desdites bactéries,
c. on dispose d'un moyen de transfert (3) entre le premier récipient (1) et le deuxième récipient (4), ledit moyen de transfert (3) comprenant au moins une première ouverture (7) au niveau du premier récipient (1) et au moins une deuxième ouverture (8) au niveau du deuxième récipient (4), de telle façon que le deuxième récipient (4) circonscrit un premier volume d'air (9) entre la deuxième ouverture (8) et le système de détection (6) desdites bactéries et/ou le moyen de transfert (3) circonscrit un deuxième volume d'air (16) entre la première ouverture (7) et la deuxième ouverture (8),
d. on applique une température T1 à l'intérieur du premier récipient (1) puis,
e. on applique une température T2 à l'intérieur du premier récipient (1)
f. la température T1 est inférieure à la température T2 de sort que l'on transfert un volume défini de milieu de culture (2) du premier récipient (1) vers le deuxième récipient (4),
g. on détermine la présence ou l'absence de bactéries et/ou on identifie les bactéries au niveau du système de détection (6).

6. Dispositif de détection et/ou d'identification de bactéries dans un échantillon comprenant
• un premier récipient (1),
• un deuxième récipient (4), comprenant au moins un système de détection (6),
• au moins un moyen de transfert (3) entre le premier récipient (1) et le deuxième récipient (4), ledit moyen de transfert comprenant au moins une première ouverture (7) au niveau du premier récipient (1) et au moins une deuxième ouverture (8) au niveau du deuxième récipient (4),
ledit deuxième récipient circonscrivant un premier volume d'air (9) entre ledit système de détection (6) et la deuxième ouverture (8) du moyen de transfert (3).

7. Dispositif selon la revendication 6 **caractérisé en ce qu'**il comprend en plus un deuxième volume d'air (16) circonscrit, au niveau du moyen de transfert (3), entre la première ouverture (7) et la deuxième ouverture (8).

8. Dispositif, selon la revendication 6 ou 7, **caractérisé en ce que** le moyen de transfert (3) est un conduit non capillaire.

9. Dispositif selon l'une quelconque des revendications 6 à 8 **caractérisé en ce que** le deuxième récipient (4) est compris dans le premier récipient (1).

10. Kit de détection et/ou d'identification de bactéries comprenant un dispositif selon l'une quelconque des revendications 6 à 9.

## Claims

1. Method for detecting and/or identifying bacteria present in a liquid or solid sample, wherein ;
a. the sample that may contain said bacteria is placed in a liquid culture medium (2), in a first container (1),
b. a second container (4) comprising at least one system (6) for detecting said bacteria is provided,
c. a means of transfer (3) between the first container (1) and the second container (4) is provided, said means of transfer (3) comprising at least a first opening (7) in the first container (1) and at least a second opening (8) in the second container (4), such that the second container (4) delimits a first volume of air (9) between the opening (8) of the transfer means (3) and the system for detecting (6) said bacteria,
d. a temperature T1 is applied inside the second container (4) then,
e. a temperature T2 is applied inside the second container (4),
f. the temperature T1 is higher than the temperature T2 such that a defined volume of culture medium (2) is transferred from the first container (1) to the second container (4),
g. the presence or absence of bacteria is determined and/or the bacteria are identified within the system for detecting said bacteria (6).

2. Method according to Claim 1, wherein the means of transfer (3) delimits a second volume of air (16) between the first opening (7) and the second opening (8).

3. Method according to Claim 1 or Claim 2, wherein T1 is between 25 and 45°C, preferably between 30 and 42°C.

4. Method according to any one of claims 1 to 3, wherein T2 is between 4 and 24°C, preferably between 13 and 18°C.

5. Method for detecting and/or identifying bacteria present in a liquid or solid sample, wherein :
a. the sample that may contain said bacteria is placed in a liquid culture medium (2), in a first container (1),
b. a second container (4) comprising at least one system (6) for detecting said bacteria is provided,
c. a means of transfer (3) between the first container (1) and the second container (4) is provided, said means of transfer (3) comprising at least a first opening (7) in the first container (1) and at least a second opening (8) in the second container (4), such that the second container (4) delimits a first volume of air (9) between the second opening (8) and the system for detecting (6) said bacteria, and/or the means of transfer (3) delimits a second volume of air (16) between the first opening (7) and the second opening (8),
d. a temperature T1 is applied inside the first container (1) then,
e. a temperature T2 is applied inside the first container (1),
f. the temperature T1 is lower than the temperature T2 such that a defined volume of culture medium (2) is transferred from the first container (1) to the second container (4),
g. the presence or absence of bacteria is determined and/or the bacteria are identified within the system for detecting said bacteria (6).

6. Device for detecting and/or identifying bacteria in a sample, comprising :
• a first container (1),
• a second container (4), comprising at least a detection system (6),
• at least a means of transfer (3) between the first container (1) and the second container (4), said means of transfer comprising at least a first opening (7) in the first container (1) and at least a second opening (8) in the second container (4), said second container delimiting a first volume of air (9) between the detection system (6) and the second opening (8) of the means of transfer (3).

7. Device according to Claim 6, wherein it also comprises a second volume of air (16) delimited, in the means of transfer (3), between the first opening (7) and the second opening (8).

8. Device, according to claim 6 or claim 7, wherein the transfer means is a non-capillary conduit.

9. Device, according to any one of the Claims 6 to 8, wherein the second container (4) is included in the first container (1).

10. Kit for detecting and/or identifying bacteria comprising a device according to any one of claims 6 to 9.

## Patentansprüche

1. Verfahren zum Nachweisen und/oder Bestimmen von in einer flüssigen oder festen Probe enthaltenen Bakterien, **dadurch gekennzeichnet, dass**
a. die möglicherweise die Bakterien enthaltende Probe in einem ersten Behälter (1) in ein flüssiges Kulturmedium (2) platziert wird,
b. ein zweiter Behälter (4), der mindestens ein System (6) zum Nachweisen der Bakterien umfasst, vorliegt,
c. ein Mittel (3) zum Transfer zwischen dem ersten Behälter (1) und dem zweiten Behälter (4) vorliegt, das mindestens eine erste Öffnung (7) im Bereich des ersten Behälters (1) und mindestens eine zweite Öffnung (8) im Bereich des zweiten Behälters (4) umfasst, so dass der zweite Behälter (4) ein erstes Luftvolumen (9) zwischen der Öffnung (8) des Transfermittels (3) und dem Bakteriennachweissystem (6) begrenzt,
d. das Innere des zweiten Behälters (4) mit einer Temperatur T1 beaufschlagt wird, dann
e. das Innere des zweiten Behälters (4) mit einer Temperatur T2 beaufschlagt wird,
f. die Temperatur T1 höher als die Temperatur T2 ist, so dass ein definiertes Volumen an Kulturmedium (2) des ersten Behälters (1) zum zweiten Behälter (4) transferiert wird, und
g. das Vorliegen oder Nichtvorliegen von Bakterien festgestellt wird und/oder die Bakterien am Nachweissystem (6) bestimmt werden.

2. Verfahren nach Anspruch 1, **dadurch gekennzeichnet, dass** das Transfermittel (3) ein zweites Luftvolumen (16) zwischen der ersten Öffnung (7) und der zweiten Öffnung (8) begrenzt.

3. Verfahren nach Anspruch 1 oder 2, **dadurch gekennzeichnet, dass** T1 zwischen 25 und 45°C, vorzugsweise zwischen 30 und 42°C, liegt.

4. Verfahren nach einem der Ansprüche 1 bis 3, **dadurch gekennzeichnet, dass** T2 zwischen 4 und 24°C, vorzugsweise zwischen 13 und 18°C, liegt.

5. Verfahren zum Nachweisen und/oder Bestimmen von in einer flüssigen oder festen Probe enthaltenen Bakterien, **dadurch gekennzeichnet, dass**
a. die möglicherweise die Bakterien enthaltende Probe in einem ersten Behälter (1) in ein flüssiges Kulturmedium (2) platziert wird,
b. ein zweiter Behälter (4), der mindestens ein System (6) zum Nachweisen der Bakterien umfasst, vorliegt,
c. ein Mittel (3) zum Transfer zwischen dem ersten Behälter (1) und dem zweiten Behälter (4) vorliegt, das mindestens eine erste Öffnung (7) im Bereich des ersten Behälters (1) und mindestens eine zweite Öffnung (8) im Bereich des zweiten Behälters (4) umfasst, so dass der zweite Behälter (4) ein erstes Luftvolumen (9) zwischen der zweiten Öffnung (8) und dem Bakteriennachweissystem (6) begrenzt, und/oder das Transfermittel (3) ein zweites Luftvolumen (16) zwischen der ersten Öffnung (7) und der zweiten Öffnung (8) begrenzt,
d. das Innere des ersten Behälters (1) mit einer Temperatur T1 beaufschlagt wird, dann
e. das Innere des ersten Behälters (1) mit einer Temperatur T2 beaufschlagt wird,
f. die Temperatur T1 niedriger als die Temperatur T2 ist, so dass ein definiertes Volumen an Kulturmedium (2) des ersten Behälters (1) zum zweiten Behälter (4) transferiert wird,
g. das Vorliegen oder Nichtvorliegen von Bakterien festgestellt wird und/oder die Bakterien am Nachweissystem (6) bestimmt werden.

6. Vorrichtung zum Nachweisen und/oder Bestimmen von Bakterien in einer Probe, mit
• einem ersten Behälter (1)
• einem zweiten Behälter (4) mit mindestens einem Nachweissystem (6) und
• mindestens einem Mittel (3) zum Transfer zwischen dem ersten Behälter (1) und dem zweiten Behälter (4) vorliegt, das mindestens eine erste Öffnung (7) im Bereich des ersten Behälters (1) und mindestens eine zweite Öffnung (8) im Bereich des zweiten Behälters (4) umfasst,
wobei der zweite Behälter ein erstes Luftvolumen (9) zwischen dem Nachweissystem (6) und der zweiten Öffnung (8) des Transfermittels (3) begrenzt.

7. Vorrichtung nach Anspruch 6, **dadurch gekennzeichnet, dass** sie ferner ein zweites Luftvolumen (16) umfasst, das im Bereich des Transfermittels (3) zwischen der ersten Öffnung (7) und der zweiten Öffnung (8) begrenzt ist.

8. Vorrichtung nach Anspruch 6 oder 7, **dadurch gekennzeichnet, dass** das Transfermittel (3) eine nicht kapillare Röhre ist.

9. Vorrichtung nach einem der Ansprüche 6 bis 8, **dadurch gekennzeichnet, dass** der zweite Behälter (4) im ersten Behälter (1) eingeschlossen ist.

10. Kit zum Nachweisen und/oder Bestimmen von Bakterien mit einer Vorrichtung nach einem der Ansprüche 6 bis 9.
